Europäisches Patentamt

European Patent Office

Office européen des brevets

(11) Veröffentlichungsnummer: **0 445 625 A1**

## (12) EUROPÄISCHE PATENTANMELDUNG

(21) Anmeldenummer: 91102804.1

(22) Anmeldetag: 26.02.91

(51) Int. Cl.5: **A61K 39/205**, A61K 39/42, C12P 21/08, C12N 15/07, G01N 33/569

Der (Die) Mikroorganismus (Mikroorganismen) ist (sind) bei der European Collection of Animal Cell Cultures unter der (den) Nummer(n) TW-1 90022604 hinterlegt worden.

(30) Priorität: 03.03.90 DE 4006630

(43) Veröffentlichungstag der Anmeldung:
11.09.91 Patentblatt 91/37

(84) Benannte Vertragsstaaten:
AT BE CH DE DK ES FR GB GR IT LI LU NL SE

(71) Anmelder: **BEHRINGWERKE Aktiengesellschaft Postfach 1140 W-3550 Marburg 1(DE)**

(72) Erfinder: **Enssle, Karlheinz, Dr. Lahnblick 14 W-3550 Marburg(DE)**
Erfinder: **Hilfenhaus, Joachim, Dr. Auf'm Gebrande 24 W-3550 Marburg(DE)**
Erfinder: **Köhler, Roland Am Ziegenberg 8 W-3550 Marburg(DE)**
Erfinder: **Kurrle, Roland, Dr. Kiefernweg 12 W-3556 Niederweimar(DE)**
Erfinder: **Seiler, Friedrich-Robert, Dr. Oberer Eichweg 10 W-3550 Marburg(DE)**

(74) Vertreter: **Klein, Otto, Dr. et al Hoechst AG Zentrale Patentabteilung Postfach 80 03 20 W-6230 Frankfurt am Main 80(DE)**

(54) Humane monoklonale Antikörper gegen Tollwutviren, ihre Herstellung und Verwendung.

(57) Die vorliegende Patentanmeldung bezieht sich auf humane monoklonale Antikörper gegen Tollwutviren und deren Verwendung in Diagnose, Prophylaxe und Therapie. Antikörper gegen das durch den humanen monoklonalen Antikörper TW-1 definierte Epitop auf dem 67 kD Glykoprotein der Virushülle schützen nach erfolgter Infektion besser gegen den Ausbruch von Tollwut als handelsübliche Präparate von humanen Immunglobulinen.

EP 0 445 625 A1

Die vorliegende Patentanmeldung bezieht sich auf humane monoklonale Antikörper gegen Tollwutviren und deren Verwendung in Diagnose, Prophylaxe und Therapie. Antikörper gegen das durch den humanen monoklonalen Antikörper TW-1 definierte Epitop auf dem 67 kD Glykoprotein der Virushülle schützen nach erfolgter Infektion besser gegen den Ausbruch von Tollwut als handelsübliche Präparate von humanen Immunglobulinen.

Bei der Tollwut handelt es sich um eine tödlich verlaufende Encephalomyelitis, die durch Tollwutvirus, ein Mitglied der Familie der Rhabdoviridae, verursacht wird. Vor allem in weniger entwickelten Ländern stellt die Erkrankung ein großes Problem dar, so werden in Indien nach vorsichtigen Schätzungen über 20000 Tote pro Jahr der Tollwut zugeschrieben. Die Ansteckung erfolgt oft über Biß durch infizierte Tiere (Canidae, Chiroptera). Erste Zielzelle für das Virus scheinen Muskelzellen an der Eintrittstelle zu sein. Nach Eintritt in periphere Nerven wandert das Virus von der Infektionsstelle über retrograden axoplasmatischen Transport zum Zentralnervensystem und Rückenmark. Nach der viralen Replikation im Zentralnervensystem wandert das Virus über die Nervenbahnen zu verschiedenen Zielgeweben im Körper, z. B. Speicheldrüsen.

Bedingt durch die relativ zu anderen Virusinfektionen lange Inkubationszeit bis zum Auftreten tollwutspezifischer Symptome von etwa 30 bis 90 Tagen ist eine postexpositionelle Impfung möglich. Um bis zum Ansprechen der aktiven Immunisierung einen schnellen Schutz zu erreichen, wird von der WHO zusammen mit der aktiven Immunisierung dringend eine passive Immunisierung mit homologen oder heterologen Serumpräparationen empfohlen In unterentwickelten Ländern werden vor allem Präparate aus Pferdeseren eingesetzt, wobei anaphylaktische Reaktionen ein ernstes Problem darstellen.

Gegenüber polyklonalen Präparaten bieten monoklonale Antikörper den Vorteil der gleichbleibenden Spezifität. Humane monoklonale Antikörper (HUMAK) vermeiden darüber hinaus unerwünschte Immunreaktionen des Empfängers gegen heterologe monoklonale Antikörper murinen Ursprungs und scheinen eine längere Zirkulationszeit im Körper zu haben.

Das Tollwutvirus ist behüllt und von geschoßförmiger Morphologie. Die Einzelstrang-RNA des Virus ist mit einem 55 kD Nukleoprotein, dem 190 kD "large transcriptase" Molekül und dem 38 kD Nicht-Strukturprotein assoziiert. Die Virushülle enthält ein 26 kD Matrixprotein und ein 67 kD Glykoprotein. Dieses Glykoprotein stellt eine wichtige Bindungsstelle für virusneutralisierende Antikörper dar (D.L. Lodmell, J. Virol., 61, 10: 3314-3318, 1987). Für den Mechanismus der Neutralisation wird eine Inhibition des intraendosomalen Fusionsschrittes vermutet, d. h. ein "uncoating" des Virus wird verhindert. Dietzschold et al. (Virology, 161: 29-36, (1987)) beschreiben die Neutralisierung von Tollwutvirus durch murine MAK gegen das 67 kD Glykoprotein. Während eine seit langem etablierte Technik (G. Köhler u. C. Milstein, Nature, 256, 495-497, 1975) es relativ einfach erlaubt, murine monoklonale Antikörper gegen bestimmte Antigene wie das Glycoprotein von Tollwutvirus zu etablieren (D.L. Lodmell, a.a.O.) ist es ausgesprochen schwierig, humane monoklonale Antikörper für die therapeutische Verwendung zu gewinnen. Es war darüber hinaus unbekannt, ob solche humanen monoklonalen Antikörper nicht nur an das Glycoprotein binden, sondern infektiöses Tollwutvirus neutralisieren und in vivo vor dem Ausbruch der Erkrankung schützen können.

Zur Gewinnung des hier beschriebenen humanen monoklonalen Antikörpers TW-1 gegen Tollwutvirus wurden humane periphere Blutlymphozyten eines serumpositiven gesunden Spenders mit Epstein-Barr-Virus (EBV) inkubiert. Über-stände auswachsender, durch EBV transformierter B-Lymphozyten wurden im ELISA auf tollwutspezifische Antikörper geprüft. Spezifisch produzierende B-Zell-Linien wurden zur Stabilisierung der Antikörperproduktion mit der Maus-Myelomzellinie SP2/O fusioniert. Die entstandenen Hybridome wurden mehrfach kloniert und auf spezifische Produktion von Antikörpern der IgG-Klasse getestet. Das Verfahren wurde nach einer bekannten Methode (D. Kozbor & J.C. Roder, Immunol. Today, 4:72-79, 1983) abgeändert und wie an anderer Stelle beschrieben (J. Hilfenhaus et al., Behring Inst. Mitt., 80:31-41, 1986) durchgeführt. Die Selektion antikörper-produzierender Zellen erfolgte dabei durch Austesten der Überstände der Kulturen im ELISA auf tollwutvirus-haltigem Beschichtungsmaterial. Der eingesetzte ELISA ist in Beispiel 1 näher erläutert.

Es wurde gefunden, daß der hier beschriebene humane monoklonale Antikörper gegen Tollwutvirus TW-1 beim Test im ELISA spezifisch mit tollwut-haltigem Beschichtungsmaterial (durchgeführt mit den Stämmen Pitman-Moore, Fluri-LEP, CVS), nicht aber mit tollwutvirus-freien Kontrollmaterialien reagiert. Ebenso wurde keine Bindung von TW-1 an Beschichtungsmaterial enthaltend Kontrollviren wie VZV, CMV, Rubella, HSV oder Masern gefunden. TW-1 bindet an tollwutvirus-infizierte, nicht aber nichtinfizierte fixierte Hühnerfibroblasten, da nach Inkubation von TW-1 mit diesen Zellen und anschließender Inkubation mit FITC-gekoppeltem Antiserum gegen humanes Immunglobulin nur auf infizierten Zellen die tollwuttypische Fluoreszenz festgestellt werden kann. Weiterhin wurde gefunden, daß TW-1 zur IgG1-Immunglobulinklasse gehört (Lambda). Nach SDS-Gelelektrophorese von mit Dithiothreitol (DTT) reduziertem Tollwutantigen (durchgeführt mit den Stämmen Pitman-Moore und Fluri-LEP) konnte im Western Blot eine Reaktion von TW-1 mit einer Bande um 67 kD gefunden werden. Aus Vergleichen der selbst gefundenen Daten mit Literaturdaten (W.H. Wunner et al., Reviews of Infectious Diseases, 10, 4:771-784, 1988) läßt sich schließen, daß es sich bei dem durch TW-1 erkannten Protein um das Glykoprotein von Tollwutvirus handelt. Bestätigt wird dies durch die Beobachtung, daß nach Verdau des Tollwutantigens mit Endoglycosidase F vor dem Auftrennen des Antigens im SDS-Gel im Western Blot eine Verringerung des Molekulargewichts des durch TW-1 markierten Proteins beobachtet werden kann. Da Endoglycosidase-F N-glykosidisch verknüpfte Zuckerreste von Proteinen abspaltet, kann aus den gewonnen Daten geschlossen werden, daß es sich bei der von TW-1 markierten Bande im Western Blot um das Glykoprotein von Tollwutvirus handelt. Die Kreuzreaktion von TW-1 mit den getesteten Tollwutvirusstämmen Pitman-Moore, Fluri-LEP und CVS deutet auf eine Bindung an ein relativ konserviertes Epitop innerhalb des Glykoproteins hin.

Die Austestung, ob TW-1 die Fähigkeit zur Neutralisation von Tollwutvirus besitzt, erfolgte nach zwei unterschiedlichen, an sich bekannten Methoden. Als erster Testtyp wurde ein "in vitro" Fluoreszenz-Neutralisationstest durchgeführt. Antikörperverdünnungen wurden mit aktiven Tollwutviren gemischt. Nach Zugabe von tollwutsuszeptiblen Zellen wurde 24 Stunden inkubiert, danach mit tollwutspezifischem FITC-gekoppeltem Antiserum inkubiert und im Fluoreszenz-Invertoskop ausgewertet. Kulturen, die tollwutspezifische Fluoreszenz zeigen, werden als positiv gewertet. Da negative Kulturen nur dann auftreten, wenn Tollwutviren vollständig durch zugegebene Antikörper neutralisiert werden, kann mit diesem Test durch geeignete Verdünnungen und Vergleich mit tollwutspezifischem Standardserum bekannter Aktivität ein Antikörper auf tollwutspezifische neutralisierende Aktivität untersucht werden. Als zweite Neutralisationstest-Methode wurde ein Versuch durchgeführt, bei dem nach dem Mischen der Antikörperverdünnungen mit infektiösem Tollwutvirus nach einer Inkubation "in vitro" das Gemisch intracerebral in NMRI-Mäuse appliziert wurde (P. Atanasiu, Laboratory Techn. in Rabies, 3rd Edition, Editors M.M. Kaplan and H. Koprowski, WHO (1973) 314-318). Werden aktive Tollwutviren durch zugegebene Antikörper vollständig neutralisiert, so ist der Anteil erkrankender Tiere in dieser Gruppe geringer als bei reiner Virusgabe ohne vorherige Antikörperzumischung. Es wurde gefunden, daß sowohl im Fluoreszenz-Neutralisationstest als auch im Neutralisationstest mit Applikation in Mäusen Tollwutvirus (Stamm CVS) durch TW-1 neutralisiert wird.

Zum Nachweis, daß die Gabe von TW-1 Antikörper in vivo bei einer Infektion mit Tollwutvirus den Ausbruch der Erkrankung hemmen oder verhindern kann, wurden Schutzversuche in NMRI-Mäusen durchgeführt. Es wurde gefunden, daß TW-1 Antikörper NMRI-Mäuse auch 2 Stunden nach der Infektion mit Tollwutviren vor dem Ausbruch der Krankheit schützen kann. Gegenüber einer Präparation aus humanen Seren mit Antikörpern gegen Tollwut (handelsüblichem Berirab[R], Behringwerke AG) wird bei Gabe von TW-1 deutlich weniger Protein für die gleiche neutralisierende und schützende Wirkung benötigt. Dies stellt einen deutlichen Vorteil von TW-1 dar.

Neben der Anwendung als Immunprophylaktikum kann der Antikörper, an geeignetes Trägermaterial gebunden, auch zur Reinigung von Tollwutviren oder Tollwutvirus-Glykoprotein über affinitätschromatographische Verfahren verwendet werden. Außerdem eignet sich der Antikörper zur Validierung des Glykoprotein-Anteils in Impfstoffpräparationen, da Tollwutvirus-Glykoprotein, wie in den Beispielen prinzipiell dargelegt, spezifisch nachgewiesen werden kann.

Die Erfindung betrifft folglich ein Epitop auf dem 67 kD Glykoprotein von Tollwutvirus, das durch den monoklonalen Antikörper TW-1 definiert ist, sowie poly- oder bevorzugt humane monoklonale Antikörper gegen vorgenanntes Epitop, ganz bevorzugt TW-1 Antikörper.

Ebenfalls umfaßt von der Erfindung ist die Verwendung vorgenannter Antikörper zur Herstellung passiver Impfstoffe, zur Diagnostik, zur Reinigung von Tollwutviren oder Tollwutvirus-Glykoprotein und zur Validierung von Impfstoffpräparationen. Die Erfindung ist schließlich in den Beispielen und den Patentansprüchen enthalten.

Das Hybridom, das den humanen monoklonalen Antikörper TW-1 sezerniert, wurde gemäß Budapester Vertrag bei der European Collection of Animal Cell Cultures (ECACC), Porton Down, Salisbury, UK am 26.02.1990 unter Nr. 90022604 hinterlegt.

**Beispiele:**

Die nachfolgenden Beispiele dienen der näheren Erläuterung der Erfindung, schränken die Erfindung jedoch nicht ein. Für die in den Beispielen geschilderten Experimente wurden affinitätschromatographisch gereinigte Antikörper eingesetzt.

**Beispiel 1: Spezifität von TW-1 Antikörpern im ELISA**

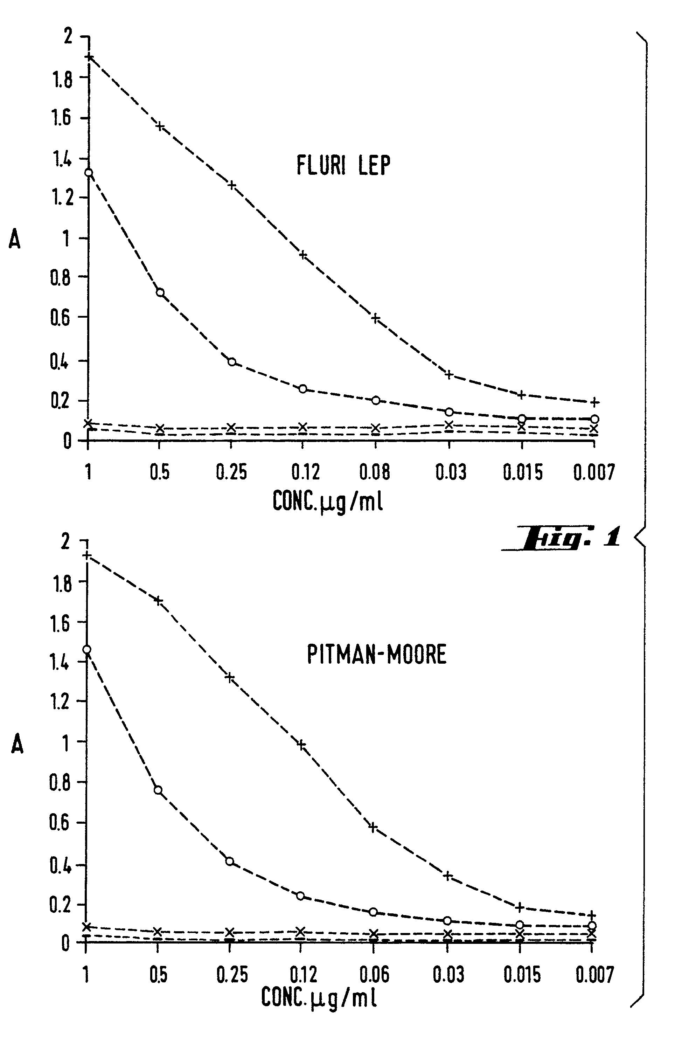

96-well ELISA-Platten wurden 24 Stunden bei Raumtemperatur mit durch Sucrose-Gradienten-Zentrifugationen gereinigtem und mittels $\beta$-Propiolacton inaktiviertem Konzentrat von Kulturüberständen infizierter MRC-5 Zellen (Stamm Pitman-Moore) bzw. infizierter Hühnerfibroblasten (Stamm Fluri-LEP) oder Präparationen der entsprechenden nichtinfizierten Zellen als Kontrolle beschichtet. Verdünnungen der Antikörperproben wurden 1 Stunde auf der Platte inkubiert und nichtgebundener Antikörper durch Waschen entfernt. Gebundene Antikörper wurden durch eine einstündige Inkubation mit einem Peroxidase-konjugierten Antikörper (Kaninchen-anti-human IgG, Behringwerke) markiert. Über eine Peroxidase-katalysierte Farbreaktion konnten humane Antikörper mit Spezifität für Tollwutvirus nachgewiesen werden. In Fig. 1 ist die Titration von TW-1 und Berirab[R] auf den aufgegebenen Tollwutantigenen und den entsprechenden Kontrollantigenen dargestellt. Werden Varicella-Zostervirus, Cytomegalovirus, Rubellaviren, Herpes-Simplex-Virus1 oder Masernviren zur Beschichtung eingesetzt, erfolgt keine Reaktion. Daher kann angenommen werden, daß TW-1 hochspezifisch an Tollwutvirus bindet. Da die Bindungskurven von TW-1 gegen die verwendeten Tollwutstämme sehr ähnlich verlaufen, kann eine annähernd gleiche Bindungsaffinität von TW-1 gegen Fluri-LEP und Pitman-Moore angenommen werden.

**Beispiel 2: Charakterisierung des Bindungsepitops von TW-1 Antikörper im Western Blot**

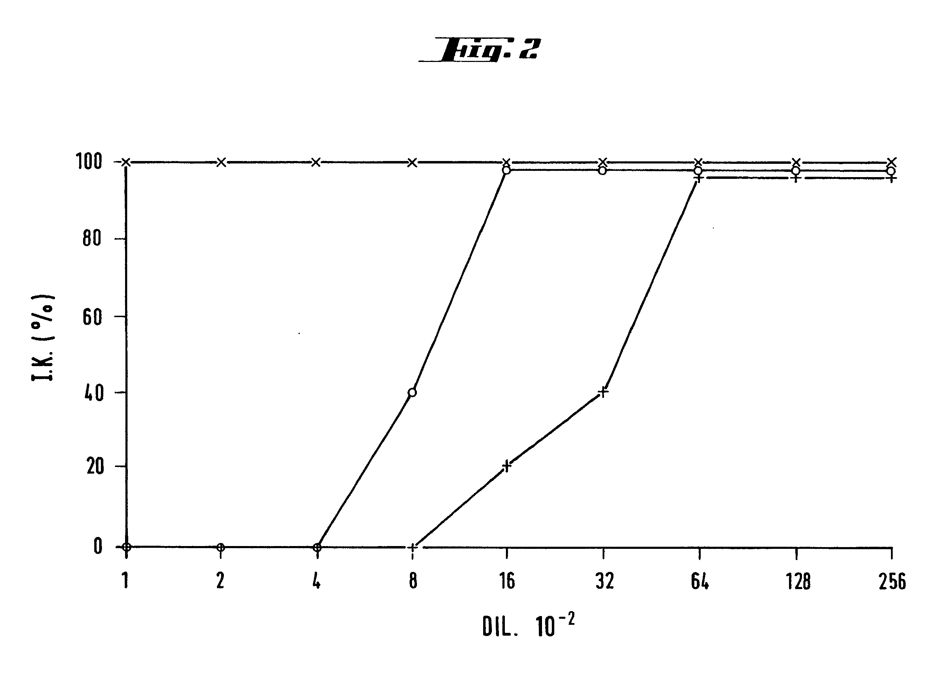

Die Auftrennung von gereinigtem Tollwutvirus (Stamm Pitman-Moore) erfolgte im 10%igen Polyacrylamidgel nach Laemmli mit 1% SDS/50 mM DTT. Nach Übertragung des aufgetrennten Antigens auf Nitrozellulose wurde mit TW-1 Antikörper, Tollwut-positivem und Tollwut-negativem Humanserum inkubiert. Der Nachweis erfolgte mit Biotin-gekoppeltem Schaf-anti-Human-Immunglobulin (Amersham), anschließender Inkubation mit Streptavidin-Peroxidase (Amersham) und Entwicklung mit Chlornaphtol (Merck). TW-1 Antikörper reagiert im Western Blot mit einer Bande um 67 kD (Fig. 2). Aus Literaturangaben (W. Wunner et al., a.a.O.) läßt sich schließen, daß es sich um das Glykoprotein von Tollwutvirus handelt. Bestätigt wird dies durch Verdau der Tollwutproteine mit Endoglycosidase F (Boehringer Mannheim) vor dem Auftragen auf das SDS-Gel. Der Verdau wurde nach den Angaben des Herstellers durchgeführt. Durch Abspaltung von Zuckerresten vom Glykoprotein sollte eine Verminderung des Moleklargewichts beobachtet werden. Eine solche Verminderung ist bei der 67 kD-Bande nach Reaktion von TW-1 Antikörper im Western-Blot im Vergleich zum ursprünglichen Antigen deutlich erkennbar und weist damit auf eine Glykosilierung des von TW-1 Antikörper erkannten Proteins hin. Keine Reaktion von TW-1 Antikörper erfolgte mit Kontrollantigenen wie CMV, Bovinem Serum Albumin oder Humanem Serum Albumin.

**Beispiel 3: In vitro-Fluoreszenz-Neutralisationstest (RFFIT)**

Der Test wurde in vitro nach der Methode von Smith et al., (J.S. Smith et al., Laboratory Techn. in Rabies, 3rd Edition, Editors M.M. Kaplan and H. Koprowsky, WHO(1973), 354-357) als Mikrotest in Mikrotiterplatten (E. Zalan, J. of Biol. Stand., 7:213-220, 1979) durchgeführt. Probenverdünnungen (Antikörper oder PBS für die Viruskontrolle) wurden mit infektiösem Tollwutvirus gemischt. Aus den durchgeführten Tests (Fig. 2) läßt sich ableiten, daß TW-1 Antikörper in diesem Test noch bei einer Konzentration von etwa 0,14 $\mu$g/ml einen neutralisierenden Effekt auf die eingesetzte Virusmenge zeigt. Vom eingesetzten Referenzserum (Pferd) werden für den gleichen Effekt etwa 13,88 $\mu$g/ml benötigt. Ein zur Kontrolle eingesetzter humaner monoklonaler Antikörper gegen Varizella Zoster (VZV-6) vom gleichen Isotyp hatte in vergleichbarer Konzentration keinen Effekt auf die Infektiosität des Virus.

**Beispiel 4: In vitro Neutralisationstest mit Inokulation des Antikörper/Antigen-Gemischs in suszeptible NMRI-Mäuse**

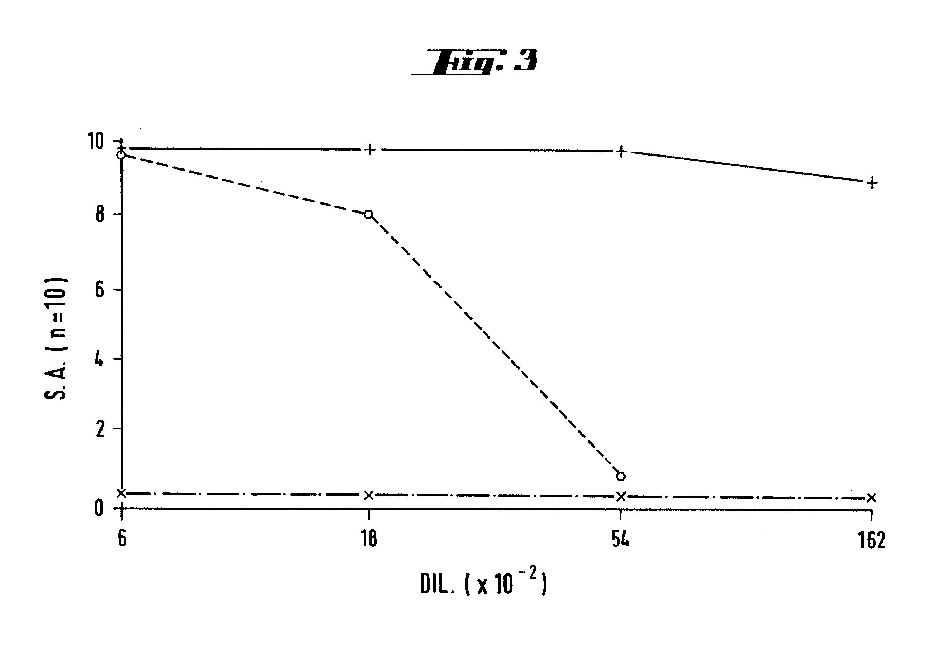

Der Test wurde nach Atanasiu (a.a.O.) durchgeführt. Zum Versuch wurden 20 g schwere NMRI-Mäuse eingesetzt. Zur Virusbelastung wurden 60 $LD_{50}$ pro Maus verwendet. Entsprechend Beispiel 3 wurden zunächst Probenverdünnungen (Antikörper oder Kontrollen) mit aktivem Tollwutvirus in vitro gemischt und inkubiert. Zur Kontrolle der Neutralisation wurde das Gemisch anschließend intracerebral in NMRI-Mäuse appliziert. Die Mäuse wurden 14 Tage täglich kontrolliert und die Zahl der überlebenden Tiere für TW-1 Antikörper, tollwutspezifisches Kontrollserum, einen VZV-spezifischen Hu-MAK und reine Virusgabe verglichen. Die entsprechenden Daten nach 14 Tagen Inkubationszeit sind in Fig. 3 dargestellt. Es wird deutlich, daß TW-1 Antikörper selbst in der geringen eingesetzten Konzentration von 0,08 $\mu$g/ml deutliche Neutralisation von Tollwutvirus zeigt, während VZV-6 in gleicher Konzentration keinerlei neutralisierenden Effekt aufweist.

**Beispiel 5: Schutzexperiment**

Der Schutztest wurde mit Tollwutvirus-suszeptiblen NMRI-Mäusen (Gewicht: 20 g) durchgeführt. Zur Virusbelastung wurden 150 $LD_{50}$ i.m. (Stamm

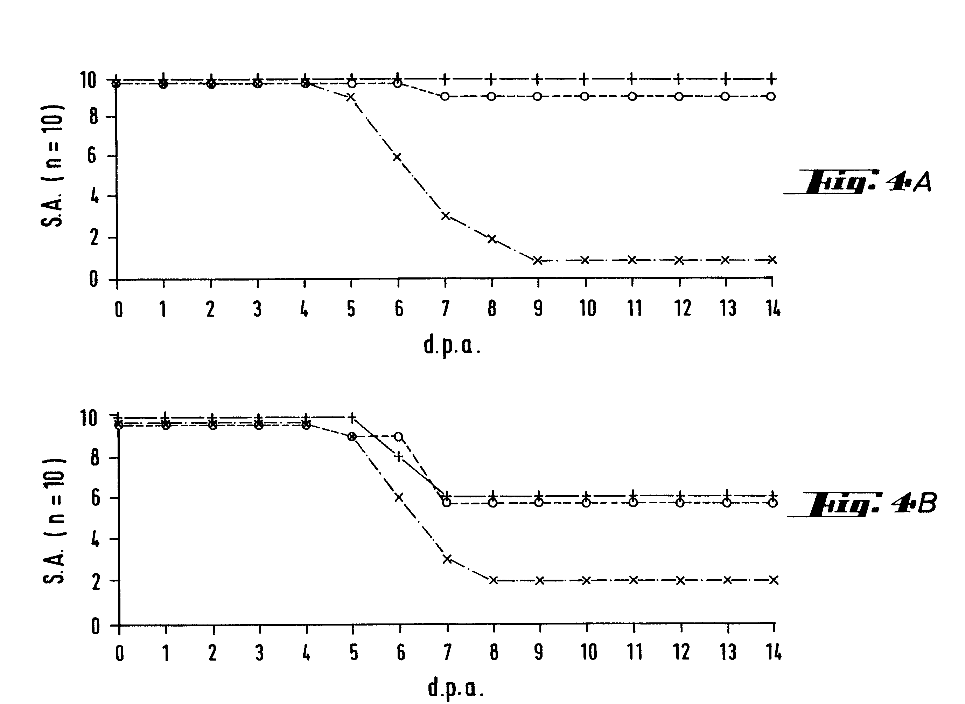

CVS) appliziert. Verdünnungen von TW-1 Antikörper, handelsüblichem Berirab^R oder von VZV-6 wurden i.v. 2 Stunden vor oder 2 Stunden nach Applikation von aktivem Tollwutvirus verabreicht. Bei dem in Fig. 4 dargestellten Experiment wurde mit 2,5 μg/ml IgG TW-1 Antikörper der gleiche Schutz erzielt wie mit 1,5mg/ml polyklonalem Berirab^R. Als Kontrolle wurde VZV-6 in doppelter Konzentration wie TW-1 Antikörper eingesetzt. Der Effekt von VZV-6 lag im Bereich der Viruskontrolle ohne Gabe von Antikörpern.

**Legende zu Fig. 1 (Beispiel 1):**

**Spezifität von TW-1 Antikörper im ELISA**

Als Anfangskonzentrationen wurden 1 μg/ml TW-1 Antikörper und 100 μg/ml IgG Berirab (Behringwerke) eingesetzt. Die Messung der Extinktion erfolgte bei 492 nm. Die Beschichtung der ELISA-Platten wurde mit 0,4 IE/ml durchgeführt. A bedeutet Extinktion, Conc. die Konzentration der eingesetzten Antikörper.
+ TW-1 auf Tollwutantigen
* TW-1 auf Kontrollantigen
O Berirab^R auf Tollwutantigen
- Berirab^R auf Kontrollantigen

**Legende zu Fig. 2 (Beispiel 3):**

**In vitro Neutralisationstest mit Fluoreszenzauswertung**

Als Anfangskonzentrationen wurden 4,4 μg/ml TW-1 Antikörper (≙ 1:100), 111,1 μg/ml (≙ 1:100) Standardserum und 3,8 μg/ml VZV-6 (≙ 1:100) eingesetzt. I.K. bedeutet infizierte Kulturen
Auf der Abszisse ist Antikörperverdünnung (Dil.) aufgetragen
+ TW-1
O Tollwut-Standard
* VZV-6

**Legende zu Fig. 3 (Beispiel 5):**

**In vitro Neutralisationstest, Auswertung in vivo**

Als Anfangskonzentrationen wurden 0,7 μg/ml TW-1 Antikörper (≙ 1:600), 18,5 μg/ml Berirab^R - (Behringwerke AG) und 0,6 μg/ml VZV-6 (≙ 1:600) eingesetzt. S.A. bedeutet: überlebende Tiere
Auf der Abszisse ist die Antikörperverdünnung (Dil.) aufgetragen
+ TW-1
O Tollwut-Standard
* VZV-6

**Legende zu Fig. 4:**

**Schutzexperimente in vivo**

Fig.4 A: Applikation der Antikörper 2 Stunden vor der Virusgabe. Fig. 4 B: Applikation der Antikörper 2 Stunden nach Virusgabe. Es wurden 2,5 μg/ml TW-1 Antikörper, 1,5 mg/ml IgG Berirab^R und 5 μg/ml VZV-6 eingesetzt.
S.A. siehe oben
d.p.a. bedeutet: Tage nach Applikation

**Patentansprüche**

1. Epitop auf dem 67 kD Glycoprotein von Tollwutvirus, dadurch gekennzeichnet, daß der humane monoklonale Antikörper sezerniert durch das Hybridom TW-1 mit der Hinterlegungs-Nr. 90022604 bei der European Collection of Animal Cell Cultures (ECACC) mit ihm reagiert.

2. Poly- oder monoklonaler Antikörper gegen das Epitop nach Anspruch 1.

3. Humaner monoklonaler Antikörper nach Anspruch 2, sezerniert von dem Hybridom mit der Hinterlegungs-Nr. 90022604 bei der ECACC.

4. Verfahren zur Herstellung von Immunprophylaktika, dadurch gekennzeichnet, daß Antikörper nach Anspruch 2 oder 3 eingesetzt werden.

5. Verfahren zum Nachweis einer Tollwutvirusinfektion, dadurch gekennzeichnet, daß Antikörper nach Anspruch 2 oder 3 eingesetzt werden.

6. Verfahren zur Validierung von Immunprophylaktika, dadurch gekennzeichnet, daß Antikörper nach Anspruch 2 oder 3 eingesetzt werden.

7. Verfahren zur Reinigung von Tollwutvirus-Antigen, dadurch gekennzeichnet, daß Antikörper nach Anspruch 2 oder 3 eingesetzt werden.

8. Zellinie oder andere Expressionssysteme, die Antikörpermoleküle nach Anspruch 2 oder Teile dieser Antikörper produziert.

9. Zellinie nach Anspruch 8, dadurch gekennzeichnet, daß sie durch Transformation von humanen Lymphozyten durch Epstein-Barr-Virus und anschließender Fusion mit einer Myelomzelle entstanden ist.

10. Zellinie TW-1 mit der Hinterlegungs-Nr. 90022604 bei der ECACC.

11. Poly- oder monoklonaler Antikörper nach Anspruch 2 oder 3 als Immunprophylaktikum oder Diagnostikum.

12. Verwendung von Antikörpern nach Anspruch 2 oder 3, zur Reinigung von Tollwutvirus-Antigen, zur Diagnostik oder zur Immunprophylaxe.

**Patentansprüche für folgenden Vertragsstaat: ES**

1. Verfahren zur Herstellung von Immunprophylaktika, dadurch gekennzeichnet, daß Antikörper eingesetzt werden, die mit einem Epitop auf dem 67 kD Glycoprotein von Tollwutvirus reagieren, wobei vorgenanntes Epitop mit dem Antikörper reagiert, der durch das bei der European Collection of Animal Cell cultures (ECACC) unter Hinterlegungs-Nr. 90022604 hinterlegte Hybridom sezerniert wird.

2. Verfahren zum Nachweis einer Tollwutvirusinfektion, dadurch gekennzeichnet, daß die in dem Verfahren nach Anspruch 1 eingesetzten Antikörper verwendet werden oder der Antikörper des Hybridoms Nr. 90022604 hinterlegt bei der ECACC zum Einsatz kommt.

3. Verfahren zur Validierung von Immunprophylaktika, dadurch gekennzeichnet, daß die in dem Verfahren nach Anspruch 1 eingesetzten Antikörper verwendet werden oder der Antikörper des Hybridoms Nr. 90022604 zum Einsatz kommt.

4. Verfahren zur Reinigung von Tollwutvirus-Antigen, dadurch gekennzeichnet, daß die in dem Verfahren nach Anspruch 1 eingesetzten Antikörper verwendet werden oder der Antikörper des Hybridoms Nr. 90022604 zum Einsatz kommt.

*Fig. 1*

_Fig. 2_

EP 0 445 625 A1

Fig. 3

EP 0 445 625 A1

*Fig. 4·A*

*Fig. 4·B*

EP 0 445 625 A1

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| P,X | IMMUNOBIOLOGY Band 181, Nr. 2/3, 1990, Seite 208; K. ENSSLE et al.: "A rabies-specific human monoclonal antibody with neutralizing activity" * Zusammenfassung * – – – | 1-12 | A 61 K 39/205 A 61 K 39/42 C 12 P 21/08 C 12 N 15/07 G 01 N 33/569 |
| P,X | JOURNAL OF GENERAL VIROLOGY Namd 71, Nr. 8, Juni 1990, Seiten 1689-1696; M. LAFON et al.: "Human monoclonal antibodies specific for the rabies virus glycoprotein and N protein" * das ganze Dokument * – – – | 1-12 | |
| P,X | JOURNAL OF VIROLOGY Band 64, Nr. 6, Juni 1990, Seiten 3087-3090; B. DIETZSCHOLD et al.: "Biological Characterization of Human Monoclonal Antibodies to Rabies Virus" * das ganze Dokument * – – – | 1-12 | |
| P,X | EP-A-0 402 029   (THE WISTAR INSTITUTE) * das ganze Dokument, insbesondere Seiten 3724-3735,3746-3754 * – – – | 1-12 | |
| X | BIOTECHNOLOGY ABSTRACTS DATABASE Zusammenfassung Nr. 89-10963 (accession Nr. 092972); Y GOZES et al.: "Establishment of a human hybridoma secreting monoclonal antibodies toward rabies virus. Human monoclonal antibody production for use in therapy" & Mod. Approaches Anim. Cell. Technol. 1987, Seiten 77-88 – – – | 1,2,4-9, 11,12 | RECHERCHIERTE SACHGEBIETE (Int. Cl.5) A 61 K 39/42 A 61 K 39/205 A 61 K 39/12 C 12 P 21/08 C 12 N 15/47 C 12 N 15/07 C 07 K 15/00 |
| Y | J. CLIN. INVEST. Band 84, Nr. 3, September 1989, Seiten 971-975; C.L. SCHUMACHER et al.: "Use of Mouse Anti-Rabies Monoclonal Antibodies in Postexposure Treatment of Rabies" * das ganze Dokument; insbesondere Seite 974 * – – – | 1-12 | |

−/−

Der vorliegende Recherchenbericht wurde für alle Patentansprüche erstellt

| Recherchenort | Abschlußdatum der Recherche | Prüfer |
|---|---|---|
| Berlin | 08 Mai 91 | JULIA P. |

**Europäisches Patentamt**

# EUROPÄISCHER RECHERCHENBERICHT

Nummer der Anmeldung

**EP 91 10 2804**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| Y | WO-A-8 909 789   (FARMITALIACARO ERBA S.R.L.) <br> * das ganze Dokument * <br> — — — | 1-12 | |
| D,A | VIROLOGY Band 161, Nr. 1, November 1987, Seiten 29-36; B. DIETZSCHOLD et al.: "Mechanisms of Rabies Virus Neutralization by Glycoprotein-Specific Monoclonal Antibodies" <br> * das ganze Dokument * <br> — — — | 1,2 | |
| Y | REVIEWS OF INFECTIOUS DISEASES Band 10, Supplement 4, November-Dezember 1988, Seiten 771-784; W.H. WUNNER et al.: "The Molecular Biology of Rabies Viruses" <br> * Seiten 776-778 * <br> — — — | 1-12 | |
| Y | EP-A-0 131 878   (SLOAN-KETTERING INSTITUTE FOR CANCER RESEARCH) <br> * das ganze Dokument * <br> — — — | 1-12 | |
| A | THE JOURNAL OF GENERAL VIROLOGY Band 48, Mai 1980, Seiten 105-109; A. FLAMAND et al.: "Use of Hybridoma Monoclonal Antibodies in the Detection of Antigenic Differences Between Rabies and Rabies-related Virus Proteins. II. The Glycoprotein" <br> * das ganze Dokument * <br> — — — — — | 1,2 | RECHERCHIERTE SACHGEBIETE (Int. Cl.5) |

Der vorliegende Recherchenbericht wurde für alle Patentansprüche erstellt

| Recherchenort | Abschlußdatum der Recherche | Prüfer |
|---|---|---|
| Berlin | 08 Mai 91 | JULIA P. |